# EUROPEAN PATENT SPECIFICATION

(11) **EP 2 468 464 B1**
(45) Date of publication and mention of the grant of the patent: **21.10.2015**
(21) Application number: 11193805.6
(22) Date of filing: 15.12.2011
(51) Int. Cl.: B25J 13/02

(54) **Apparatus and method for transmitting force or force vector**
Vorrichtung und Verfahren zur Übertragung einer Kraft oder eines Kraftvektors
Appareil et procédé de transmission de force ou de vecteur force

(30) Priority: 21.12.2010 KR 20100131339
(43) Date of publication of application: 27.06.2012
(73) Proprietor: Samsung Electronics Co., Ltd., Suwon-si, Gyeonggi-do, 443-742 (KR)
(72) Inventor: Lee, Hyung Kew, Gyeonggi-do 446-712 (KR); Park, Joon Ah, Gyeonggi-do 446-712 (KR)
(74) Representative: Grootscholten, Johannes A.M.

(56) References cited:
- JP-A- 3 270 886
- JP-A- 2003 308 141
- JP-A- 2004 029 999
- US-A- 6 126 373
- US-A1- 2008 167 662

## Description

### BACKGROUND

### 1. Field

The present invention relates to an apparatus and method for transferring a force or force vector to a human, and more particularly, to an apparatus and method, including actuators to transfer force from a plurality of directions to an object, such as a human's finger tip, inserted into the apparatus.

Such an apparatus and method are known from JP-A-2004029999

### 2. Description of the Related Art

To remotely manipulate a robot, or to intuitively manipulate an object within a virtual space, there is a need to improve the artificial generation of a a sense substantially perceived at a hand or an arm of a user when manipulating an object, and to improve the transfer of the generated sense to the user.

### SUMMARY

The foregoing and/or other aspects are achieved by providing an apparatus according to claim 1 and a method according to claim 16.

According to example embodiments, it is possible to transfer, to an end portion of a finger of a human being, a force from a plurality of directions acting on an end portion of a robot finger.

According to example embodiments, since an end portion of a robot manipulating a sensitive tissue or object, such as an operation robot, is enabled to transfer, to a manipulator, a force in contact with the tissue, it is possible to enhance the efficiency and the stability of a task using the robot.

According to example embodiments, it is possible to enhance a sensitivity of a tactile sense by transferring a force to a finger through an inner wall formed as a protrusion.

According to example embodiments, since fluid is supplied to an actuator at a predetermined pressure and is expanded by the pressure of the supplied fluid, it is possible to directly transfer a magnitude of the force to a finger.

Additional aspects of embodiments will be set forth in part in the description which follows and, in part, will be apparent from the description, or may be learned by practice of the disclosure.

### BRIEF DESCRIPTION OF THE DRAWINGS

These and/or other aspects will become apparent and more readily appreciated from the following description of embodiments, taken in conjunction with the accompanying drawings of which:
FIG. 1 illustrates an example of a plurality of forces acting on a robot finger;
FIG. 2 illustrates a perspective view of a force vector transferring apparatus according to example embodiments;
FIG. 3 illustrates an actuator included in a force vector transferring apparatus according to example embodiments;
FIG. 4 illustrates a cross-sectional view of the force vector transferring apparatus cut based on B of FIG. 2 according to example embodiments;
FIG. 5 illustrates a cross-sectional view of the force vector transferring apparatus cut based on A of FIG. 2 according to example embodiments; and
FIG. 6 illustrates a method of transferring a force vector according to example embodiments.

### DETAILED DESCRIPTION

Reference will now be made in detail to embodiments, examples of which are illustrated in the accompanying drawings, wherein like reference numerals refer to the like elements throughout. Embodiments are described below to explain the present disclosure by referring to the figures.

To remotely manipulate a robot, or to intuitively manipulate an object within a virtual space, there is a need to artificially generate a sense substantially perceived at a hand or an arm of a user when manipulating an object, and to transfer the generated sense to the user.

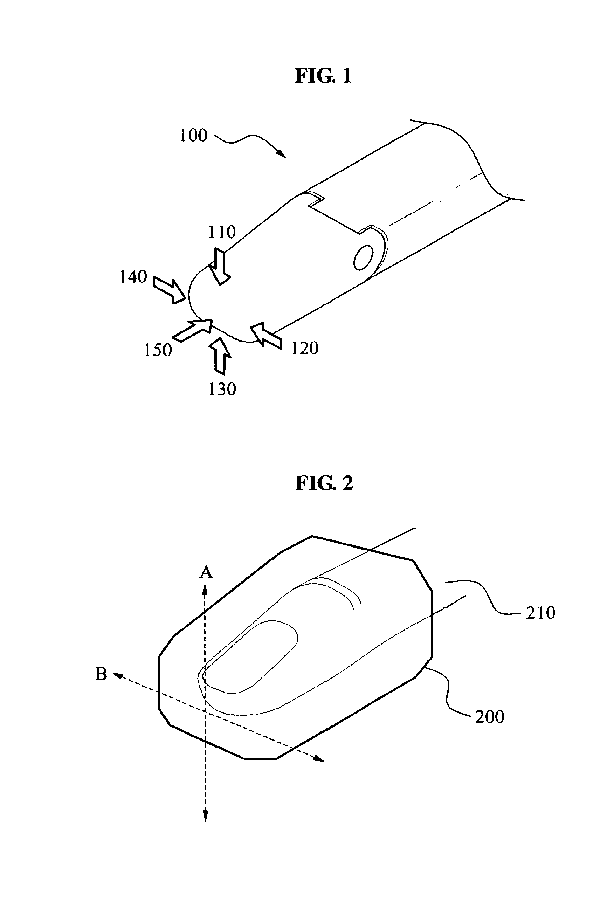

FIG. 1 illustrates an example of a plurality of forces acting on a robot finger 100.

Referring to FIG. 1, when a robot manipulates an object, a force from a plurality of directions may act on an end portion of a robot finger 100. For example, the plurality of directions may include a front direction 150, an up direction 110, a down direction 130, a left direction 140, and a right direction 120.

FIG. 2 illustrates a force vector transferring apparatus 200 according to example embodiments.

Referring to FIG. 2, the force vector transferring apparatus 200 includes an inner wall to be inserted with an object 210 and thereby be expanded by the inserted object 210, a hard outer wall to surround the inner wall in a thimble shape, and a plurality of actuators being disposed between the inner wall and the outer wall to transfer a force to the object 210 through the inner wall. The object 210 may be, for example, a finger of a human being.

The force vector transferring apparatus 200 may be attached to an end portion of a finger of a human being and thereby be used. The force vector transferring apparatus 200 may be provided in a thimble shape to transfer the force from the plurality of forces as shown in FIG. 1.

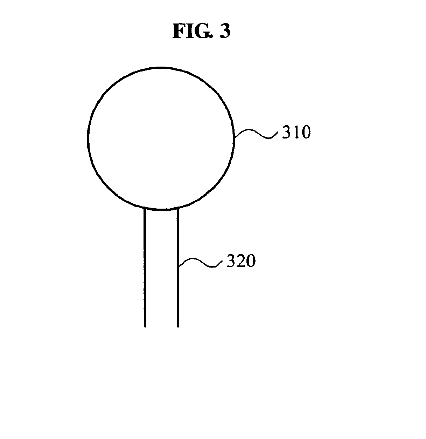

FIG. 3 illustrates an actuator 310 included in a force vector transferring apparatus 200 according to example embodiments.

Referring to FIG. 3, the actuator 310 may transfer, to an object, a force from each of a plurality of forces as shown in FIG. 1. The actuator 310 may have an expansion and contraction enabling structure. For example, the actuator 310 may be provided in a pocket structure such as a balloon formed of a flexible rubber material and containing a fluid. The expansion and the contraction of the balloon may be controlled, by a controlling module, based on an amount of fluids supplied to the balloon. A conduit 320 may be provided to supply the fluid. For example, when a fluid such as air is injected into the conduit 320 at a predetermined pressure, the balloon may be expanded by the pressure. When a deflation is performed, the balloon may be contracted.

According to an embodiment, a plurality of actuators may be provided to the force vector transferring apparatus 200 to correspond to at least one of a plurality of directions, for example, the front direction 150, the up direction 110, the down direction 130, the left direction 140, and the right direction 120 of FIG. 1. For example, five actuators may be provided to correspond to the front direction 150, the up direction 110, the down direction 130, the left direction 140, and the right direction 120.

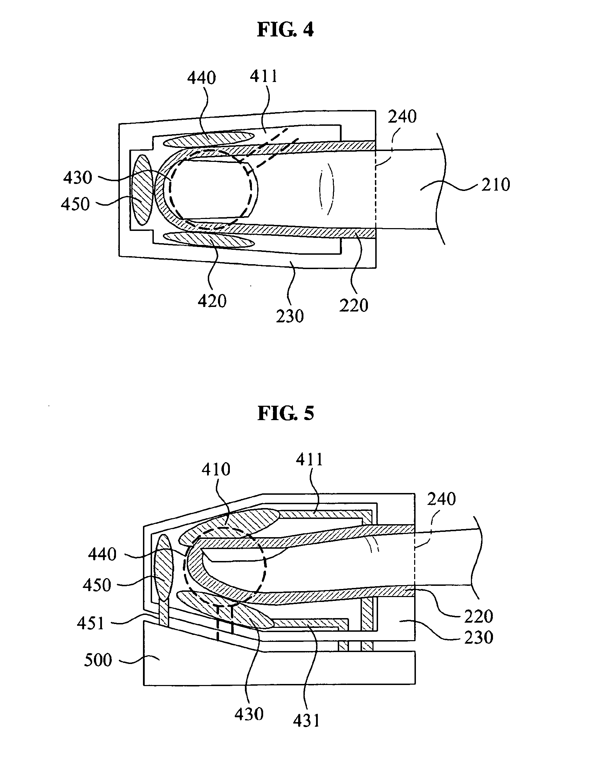

FIG. 4 illustrates a cross-sectional view of the force vector transferring apparatus 200 cut based on B of FIG. 2.

Referring to FIG. 4, the force vector transferring apparatus 200 includes an outer wall 230, an inner wall 220 to surround the object 210, and a plurality of actuators 410, 420, 430, 440, and 450.

The outer wall 230 may have a thimble shape and be formed of a hardened material. An opening 240 may be formed in the outer wall 230, and may have a size enabling the object 210, for example, an end portion of a finger of a human being, to be inserted. An inner space connected to the opening 240 may be formed to have a size sufficient to receive the end portion of the finger. As described above, the opening 240 may correspond to a space surrounded by the inner wall 220. The outer wall 230 may be provided in the thimble shape in order to be more effectively attached to the object 210, for example, the finger of the human being.

The inner wall 220 may be expanded by the inserted object 210. The inner wall 220 may make direct contact with the object 210 and thus, may be formed of a flexible material so that the force vector transferring apparatus 200 may be closely attached to the object 210.

The plurality of actuators 410, 420, 430, 440, and 450 are disposed between the inner wall 210 and the outer wall 230 to transfer the force to the object 210 through the inner wall 220. For example, five actuators 410, 420, 430, 440, and 450 may be provided to correspond to the front direction 150, the up direction 110, the down direction 130, the left direction 140, and the right direction 120 of FIG. 1.

For example, the actuator 430 corresponding to a force from the down direction 130 of the robot finger 100 may be disposed below the finger of the human being. A conduit 431 supplying a fluid to the actuator 430 may be connected below the force vector transferring apparatus 200 through an empty space formed between the inner wall 220 and the outer wall 230. The actuator 450 corresponding to the force from the front direction 150 of the robot finger 100 may be disposed in front of the finger of the human being. A conduit 451 of FIG. 5 supplying a fluid to the actuator 450 may be connected below the force vector transferring apparatus 200 through an empty space formed between the inner wall 220 and the outer wall 230.

FIG. 5 illustrates a cross-sectional view of the force vector transferring apparatus 200 cut based on A of FIG. 2.

A basic configuration will be described with reference to FIG. 5.

Conduits 411 through 451 corresponding to the actuators 410, 420, 430, 440, and 450, respectively, may be connected to a control module 500 controlling a fluid through a bottom surface of the force vector transferring apparatus 200.

When the object 210 is inserted into the inner wall 220 of the force vector transferring apparatus 200, the inner wall 220 may make direct or close contact with the object 210. The control module 500 may supply the fluid to the actuators 410, 420, 430, 440, and 450 through each of the respective conduits 411 through 451 in charge of corresponding directions. When the fluid is supplied to the actuators 410, 420, 430, 440, and 450, the pressure may be applied to the actuators 410, 420, 430, 440, and 450, whereby the actuators 410, 420, 430, 440, and 450 may transfer a force from corresponding directions to corresponding portions of the object 210.

Depending on embodiments, the force vector transferring apparatus 200 may be provided to correspond to various sizes and shapes of a finger occurring due to the flexibility of the inner wall 200 and the actuators 410, 420, 430, 440, and 450.

When a protrusion is formed in a portion where the object 210 makes direct contact with the inner wall 220 of the force vector transferring apparatus 200, it is possible to enhance the sensitivity of tactile sense of the object 210, for example, the finger.

In addition to a balloon typed actuator, it is possible to employ an actuator using an electro active polymer (EAP) or a piezoelectric element with respect to the actuators 410, 420, 430, 440, and 450.

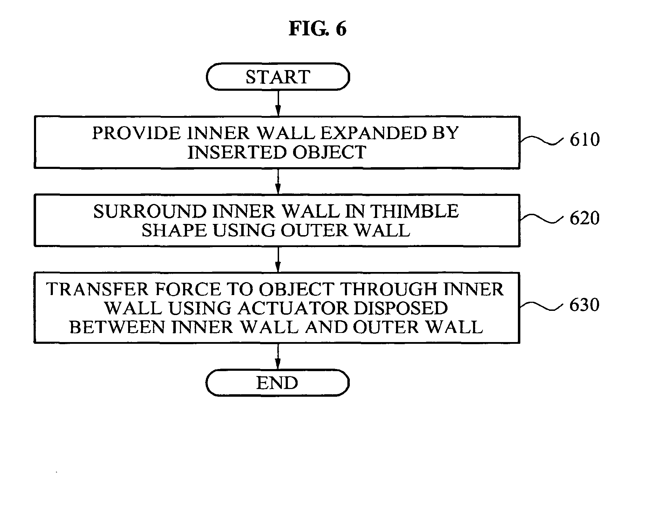

FIG. 6 illustrates a method of transferring a force vector according to example embodiments.

In operation 610, the force vector transferring apparatus 200 may provide the inner wall 220 expanded by the inserted object 210. The inner wall 220 corresponds to a portion making direct contact with the object 210 and thus, may be formed of a flexible material so that the force vector transferring apparatus 200 may be closely attached to the object 210.

In operation 620, the force vector transferring apparatus 200 may surround the inner wall 220 using the outer wall 230 formed of a hardened material.

In operation 630, the force vector transferring apparatus 200 may transfer the force to the object 210 using the actuators 410 through 450 that are disposed between the inner wall 220 and the outer wall 230. As described, the actuators 410 through 450 may be provided to correspond to the front direction 150, the up direction 110, the down direction 130, the left direction 140, and the right direction 120, respectively.

For example, when the object 210 is a finger of a human being, and when the object 210 is inserted into the inner wall 220 of the force vector transferring apparatus 200, the inner wall 220 may make direct or close contact with the finger. The control module 500 may supply the fluid to the actuators 410, 420, 430, 440, and 450 through the conduits 411 through 451 in charge of corresponding directions. When the fluid is supplied to the actuators 410, 420, 430, 440, and 450, the pressure may be applied to the actuators 410, 420, 430, 440, and 450, whereby the actuators 410, 420, 430, 440, and 450 may transfer a force from corresponding directions to corresponding portions.

By forming a protrusion in a portion where the object 210 makes direct or close contact with the inner wall 220 of the force vector transferring apparatus 200, it is possible to enhance the sensitivity of tactile sense of the object 210, for example, the finger.

Methods and apparatuses according to example embodiments may be embodied using various types of packages. For example, the methods and apparatuses may be embodied using packages such as Package on Packages (PoPs), Ball Grid Arrays (BGAs), Chip Scale Packages (CSPs), Plastic Leaded Chip Carrier (PLCC), Plastic Dual In-Line Package (PDIP), Die in Waffle Pack, Die in Wafer Form, Chip On Board (COB), Ceramic Dual In-Line Package (CERDIP), Plastic Metric Quad Flat Pack (MQFP), Quad Flatpack (QFP), Small Outline Integrated Circuit (SOIC), Shrink Small Outline Package (SSOP), Thin Small Outline (TSOP), Thin Quad Flatpack (TQFP), System In Package (SIP), Multi Chip Package (MCP), Wafer-level Fabricated Package (WFP), Wafer-Level Processed Stack Package (WSP), and the like.

The embodiments can be implemented in computing hardware (computing apparatus) and/or software, such as (in a non-limiting example) any computer that can store, retrieve, process and/or output data and/or communicate with other computers. The results produced can be displayed on a display of the computing hardware. A program/software implementing the embodiments may be recorded on non-transitory computer-readable media comprising computer-readable recording media. Examples of the computer-readable recording media include a magnetic recording apparatus, an optical disk, a magneto-optical disk, and/or a semiconductor memory (for example, RAM, ROM, etc.). Examples of the magnetic recording apparatus include a hard disk device (HDD), a flexible disk (FD), and a magnetic tape (MT). Examples of the optical disk include a DVD (Digital Versatile Disc), a DVD-RAM, a CD-ROM (Compact Disc - Read Only Memory), and a CD-R (Recordable)/RW.

Although embodiments have been shown and described, it would be appreciated by those skilled in the art that changes may be made in these embodiments without departing from the scope which is defined by the claims. For instance, different types of actuators may be used in one apparatus according to the present invention.

## Claims

1. An apparatus for transferring a force to an object, comprising:
an outer wall (230) defining an inner space and comprising an opening for an object (210) to be inserted therein; and
a plurality of actuators (410, 420, 430, 440, 450) being disposed within the outer wall to transfer forces from a plurality of directions to the object;
**characterized in that** the apparatus comprises an inner wall (220), the actuators being arranged between the inner and outer wall, and **in that** each of the plurality of actuators (410, 420, 430, 440, 450) is expanded in correspondence with a magnitude of a force to be transferred to the object (210) from each of the plurality of different directions, corresponding with each of the plurality of actuators (410, 420, 430, 440, 450) so as to directly transfer, to the object, a magnitude of the force transferred via the actuator (410, 420, 430, 440, 450).

2. Apparatus as claimed in claim 1, comprising a hard outer wall to surround the inner wall.

3. The apparatus of claims 1 or 2, wherein the inner wall is made of flexible material so as to closely attach the apparatus to the object.

4. The apparatus of any of the preceding claims, comprising a protrusion in a portion where the object (210) operationally makes direct or close contact with the inner wall (22).

5. The apparatus of any of claims 1-4, comprising a hard outer wall to surround the inner wall in a thimble shape.

6. The apparatus of claim 1, wherein the actuator (410, 420, 430, 440, 450) has an expansion and contraction enabling structure.

7. The apparatus of claim 6, wherein the actuator (410, 420, 430, 440, 450) comprises a balloon formed of a rubber material, and further a conduit to supply a fluid to the balloon.

8. The apparatus of claim 7, wherein the actuator (410, 420, 430, 440, 450) further comprises a control module (500) to control the fluid supplied to the conduit.

9. The apparatus of claim 7 or 8, wherein each of the at least one actuator (410, 420, 430, 440, 450) is supplied with a fluid at a predetermined pressure through the conduit to transfer the force using the pressure of the supplied fluid.

10. The apparatus of a preceding claim, wherein the at least one actuator (410, 420, 430, 440, 450) comprises an electro active polymer (EAP) or a piezoelectric element.

11. The apparatus of a preceding claim, comprising a plurality of actuators (410, 420, 430, 440, 450) to transfer, to the object, force from a plurality of directions.

12. The apparatus of claim 11, wherein at least five actuators (410, 420, 430, 440, 450) are provided.

13. The apparatus of claim 12, wherein the different directions comprise an up direction, a down direction, a left direction, a right direction, and a front direction relative to the object.

14. The apparatus of a preceding claim, wherein the object to be inserted is an end portion of a finger.

15. The apparatus of claim 14, wherein the inner wall is expanded based on the magnitude of the force, and stimulates a tactile sense of the end portion of the finger using a portion formed as a protrusion.

16. A method of transferring a force vector, comprising:
providing an outer wall (230) and an inner wall (220), the inner wall defining an inner space and comprising an opening for an object (210) to be inserted therein;
transferring a force to the inserted object by expanding or contracting a plurality of actuators (410 420, 430, 440, 450) disposed between the outer and inner wall, in correspondence with a magnitude of the force to be transferred to the object (210) from each of a plurality of different directions.

## Patentansprüche

1. Vorrichtung zur Übertragung einer Kraft auf ein Objekt, umfassend:
eine Außenwand (230), die einen Innenraum definiert und eine Öffnung für ein Objekt (210) umfasst, welches dort eingeführt wird; und
eine Mehrzahl von Aktuatoren (410, 420, 430, 440, 450), die so in der Außenwand angeordnet sind, dass sie Kräfte aus einer Mehrzahl von Richtungen auf das Objekt übertragen;
**dadurch gekennzeichnet, dass** die Vorrichtung eine Innenwand (220) umfasst, wobei die Aktuatoren zwischen der Innen- und der Außenwand ausgerichtet sind, und dadurch, dass jeder aus der Mehrzahl von Aktuatoren (410, 420, 430, 440, 450) in Kommunikation mit der Stärke einer Kraft ausgedehnt ist, um diese von jedem aus der Mehrzahl von Aktuatoren auf das Objekt (210) zu übertragen, in Übereinstimmung mit jedem aus der Mehrzahl von Aktuatoren (410, 420, 430, 440, 450), so dass die Stärke der durch die Aktuatoren (410, 420, 430, 440, 450) übertragenen Kraft direkt auf das Objekt übertragen wird.

2. Vorrichtung gemäß Anspruch 1, die eine harte Außenwand umfasst, welche die Innenwand umgibt.

3. Vorrichtung gemäß Anspruch 1 oder 2, wobei die Innenwand aus einem flexiblen Material besteht, damit sie die Vorrichtung dicht mit dem Objekt in Verbindung bringen kann.

4. Vorrichtung gemäß einem der vorangegangenen Ansprüche, die einen Vorsprung in einem Abschnitt umfasst, an dem das Objekt (210) bei Betrieb direkten oder dichten Kontakt mit der Innenwand (220) herstellt.

5. Vorrichtung gemäß einem der Ansprüche 1 bis 4, die eine harte Außenwand umfasst, welche die Innenwand wie ein Fingerhut umgibt.

6. Vorrichtung gemäß Anspruch 1, wobei der Aktuator (410, 420, 430, 440, 450) eine Struktur aufweist, die ein Ausdehnen und ein Zusammenziehen ermöglicht.

7. Vorrichtung gemäß Anspruch 6, wobei der Aktuator (410, 420, 430, 440, 450) einen Ballon, der aus einem Gummimaterial besteht, und weiterhin eine Rohrleitung, um dem Ballon ein Fluid zuzuführen, umfasst.

8. Vorrichtung gemäß Anspruch 7, wobei der Aktuator (410, 420, 430, 440, 450) weiterhin ein Steuermodul (500) umfasst, um das der Rohrleitung zugeführte Fluid zu steuern.

9. Vorrichtung gemäß Anspruch 7 oder 8, wobei jedem des wenigstens einen Aktuators (410, 420, 430, 440, 450) ein Fluid mit einem vorbestimmten Druck durch die Rohrleitung zugeführt wird, um die Kraft mit Hilfe des Drucks des zugeführten Fluids zu übertragen.

10. Vorrichtung gemäß einem der vorangegangenen Ansprüche, wobei jeder des wenigstens einen Aktuators (410, 420, 430, 440, 450) ein elektroaktives Polymer (EAP) oder ein piezoelektrisches Element umfasst.

11. Vorrichtung gemäß einem der vorangegangenen Ansprüche, die eine Mehrzahl von Aktuatoren (410, 420, 430, 440, 450) umfasst, um Kraft aus einer Mehrzahl von Richtungen auf das Objekt zu übertragen.

12. Vorrichtung gemäß Anspruch 11, wobei wenigstens fünf Aktuatoren (410, 420, 430, 440, 450) zur Verfügung gestellt werden.

13. Vorrichtung gemäß Anspruch 12, wobei die verschiedenen Richtungen die folgenden umfassen: nach oben, nach unten, nach links und nach rechts sowie eine frontale Richtung relativ zu dem Objekt.

14. Vorrichtung gemäß einem der vorangegangenen Ansprüche, wobei es sich bei dem Objekt, das dort eingeführt wird, um eine Fingerspitze handelt.

15. Vorrichtung gemäß Anspruch 14, wobei die Innenwand sich basierend auf der Stärke der Kraft ausdehnt und den Tastsinn der Fingerspitze mit Hilfe eines als Vorsprung geformten Abschnitts stimuliert.

16. Verfahren zur Übertragung eines Kraftvektors, umfassend:
zur Verfügung stellen einer Außenwand (230) und einer Innenwand (220), wobei die Innenwand einen Innenraum definiert und eine Öffnung für ein Objekt (210) umfasst, welches dort eingeführt wird;
Übertragen einer Kraft auf das eingeführte Objekt durch Ausdehnen oder Zusammenziehen einer Mehrzahl von Aktuatoren (410, 420, 430, 440, 450), die zwischen der Innen- und der Außenwand ausgerichtet sind, in Übereinstimmung mit der Stärke der Kraft, die auf das Objekt (210) zu übertragen ist und die aus jeder Richtung aus einer Mehrzahl von verschiedenen Richtungen kommt.

## Revendications

1. Appareil permettant de transférer une force sur un objet, comprenant :
une paroi extérieure (230) définissant un espace intérieur et comprenant une ouverture destinée à y introduire un objet (210) ; et
une pluralité d'actionneurs (410, 420, 430, 440, 450) disposés à l'intérieur de la paroi extérieure afin de transférer sur l'objet des forces en provenance d'une pluralité de directions ;
**caractérisé en ce que** l'appareil comprend une paroi intérieure (220), les actionneurs étant agencés entre la paroi intérieure et la paroi extérieure, et **en ce que** chaque actionneur de la pluralité d'actionneurs (410, 420, 430, 440, 450) est dilaté en correspondance avec la magnitude d'une force à transférer sur l'objet (210) en provenance de chaque direction parmi la pluralité de directions différentes, en correspondance avec chaque actionneur de la pluralité d'actionneurs (410, 420, 430, 440, 450) de manière à transférer directement sur l'objet une magnitude de la force transférée par le biais de l'actionneur (410, 420, 430, 440, 450).

2. Appareil selon la revendication 1, comprenant une paroi extérieure rigide qui entoure la paroi intérieure.

3. Appareil selon la revendication 1 ou 2, dans lequel la paroi intérieure est réalisée dans une matière souple de manière à ce que l'appareil épouse intimement l'objet.

4. Appareil selon l'une quelconque des revendications précédentes, comprenant une saillie dans une partie où l'objet (210) exerce un contact opérationnel direct ou étroit avec la paroi intérieure (220).

5. Appareil selon l'une quelconque des revendications 1 à 4, comprenant une paroi extérieure rigide qui entoure la paroi intérieure à la manière d'un dé à coudre.

6. Appareil selon la revendication 1, dans lequel l'actionneur (410, 420, 430, 440, 450) présente une structure permettant la dilatation et la contraction.

7. Appareil selon la revendication 6, dans lequel l'actionneur (410, 420, 430, 440, 450) comprend un ballon constitué d'une matière caoutchouteuse, et comprend en outre une conduite permettant d'apporter un fluide au ballon.

8. Appareil selon la revendication 7, dans lequel l'actionneur (410, 420, 430, 440, 450) comprend en outre un module de régulation (500) destiné à réguler le fluide apporté à la conduite.

9. Appareil selon la revendication 7 ou 8, dans lequel chacun des au moins un actionneur (410, 420, 430, 440, 450) est pourvu en fluide à une pression prédéterminée par le biais de la conduite afin de transférer la force au moyen de la pression du fluide apporté.

10. Appareil selon l'une quelconque des revendications précédentes, dans lequel l'au moins un actionneur (410, 420, 430, 440, 450) comprend un polymère électroactif (PEA) ou un élément piézoélectrique.

11. Appareil selon l'une quelconque des revendications précédentes, comprenant une pluralité d'actionneurs (410, 420, 430, 440, 450) destinés à transférer à l'objet une force en provenance d'une pluralité de directions.

12. Appareil selon la revendication 11, dans lequel il est prévu au moins cinq actionneurs (410, 420, 430, 440, 450).

13. Appareil selon la revendication 12, dans lequel les directions différentes comprennent une direction vers le haut, une direction vers le bas, une direction vers la gauche, une direction vers la droite, et une direction vers l'avant par rapport à l'objet.

14. Appareil selon l'une quelconque des revendications précédentes, dans lequel l'objet à introduire est la partie terminale d'un doigt.

15. Appareil selon les revendications 14, dans lequel la paroi intérieure est dilatée en fonction de la magnitude de la force, et stimule le sens du toucher de la partie terminale du doigt au moyen d'une partie ayant une forme en saillie.

16. Procédé de transfert d'un vecteur de force, comprenant :
la disposition d'une paroi extérieure (230) et d'une paroi intérieure (220), la paroi intérieure définissant un espace intérieur et comprenant une ouverture destinée à y introduire un objet (210) ;
le transfert d'une force sur l'objet introduit sous l'effet de la dilatation ou de la contraction d'une pluralité d'actionneurs (410, 420, 430, 440, 450) disposés entre la paroi extérieure et la paroi intérieure, en correspondance avec la magnitude de la force à transférer sur l'objet (210) en provenance de chaque direction parmi une pluralité de directions différentes.
